Europäisches Patentamt

(19) European Patent Office

Office européen des brevets

(11) Publication number: **0 127 284**
**B1**

(12) **EUROPEAN PATENT SPECIFICATION**

(45) Date of publication of patent specification: 05.08.87

(21) Application number: 84302032.2

(22) Date of filing: 27.03.84

(51) Int. Cl.⁴: **C 07 C 2/12,** C 07 C 15/02 //
C10L1/04, B01J29/28

(54) Exothermic hydrocarbon conversion system utilizing heat exchange between reactor effluent fractionation system and feedstock.

(30) Priority: 26.04.83 US 488845

(43) Date of publication of application:
05.12.84 Bulletin 84/49

(45) Publication of the grant of the patent:
05.08.87 Bulletin 87/32

(84) Designated Contracting States:
BE DE FR GB IT NL

(56) References cited:
EP-A-0 016 494
EP-A-0 031 675
EP-A-0 099 701
GB-A-1 582 820
US-A-1 843 880
US-A-4 242 531
US-A-4 433 185

(73) Proprietor: MOBIL OIL CORPORATION
150 East 42nd Street
New York New York 10017 (US)

(72) Inventor: Marsh, Susan Kim
Rd No. 1, Box 1228
Mount Holly, NJ 08060 (US)
Inventor: Owen, Hartley
5 Riverview Terrace
Belle Mead, NJ 08502 (US)
Inventor: Wright, Bernard Stanley
13 Shagbark Lane
East Windsor, NJ 08520 (US)

(74) Representative: Cooper, John Anthony et al
Mobil Court 3 Clements Inn
London WC2A 2EB (GB)

Courier Press, Leamington Spa, England.

## 0 127 284

**Description**

This invention relates to thermal exchange processes and apparatus for converting olefins to higher hydrocarbons, such as gasoline-range or distillate-range fuels. In particular it relates to techniques for operating an exothermic catalytic reactor system and effluent fractionation system to provide efficient use of thermal energy.

Developments in zeolite catalysts and hydrocarbon conversion processes have created interest in utilizing olefinic feedstocks, such as petroleum refinery streams rich in lower olefins, for producing $C_5^+$ gasoline, diesel fuel. In addition to the basic work derived from ZSM-5 type zeolite catalysts, a number of discoveries have contributed to the development of a new industrial process, known as Mobil Olefins to Gasoline/Distillate ("MOGD"). This process has significance as a safe, environmentally acceptable technique for utilizing refinery streams that contain lower olefins, especially $C_2$—$C_5$ alkenes. This process may supplant conventional alkylation units. In U.S. Patents 3,960,978 and 4,021,502, Plank, Rosinski and Givens disclose conversion of $C_2$—$C_5$ olefins, alone or in admixture with paraffinic components, into higher hydrocarbons over crystalline zeolites having controlled acidity. Garwood et al have also contributed improved processing techniques to the MOGD system, as in U.S. Patents, 4,150,062, 4,211,640 and 4,227,992.

Conversion of lower olefins, especially propene and butenes, over H-ZSM-5 is effective at moderately elevated temperatures and pressures. The conversion products are sought as liquid fuels, especially the $C_5^+$ aliphatic and aromatic hydrocarbons. Olefinic gasoline is produced in good yield by the MOGD process and may be recovered as a product or recycled to the reactor system for further conversion to distillate-range products.

Olefinic feedstocks may be obtained from various sources, including fossil fuel processing streams, such as gas separation units, cracking of $C_2^+$ hydrocarbons, coal byproducts, and various synthetic fuel processing streams. Cracking of ethane and conversion of conversion effluent is disclosed in U.S. Patent 4,100,218 and conversion of ethane to aromatics over Ga-ZSM-5 is disclosed in U.S. Patent 4,350,835. Olefinic effluent from fluidized catalytic cracking of gas oil or the like is a valuable source of olefins, mainly $C_3$—$C_4$ olefins, suitable for exothermic conversion according to the present MOGD process.

It is an object of the present invention to improve process economics by lowering equipment costs and increasing thermal efficiency in an olefin oligomerization system. This is achieved by a technique employing a heat exchange sequence wherein hot reactor effluent is first separated into a light hydrocarbon stream ($C_4^-$) and heavier hydrocarbon stream ($C_5^+$) from which distillate and gasoline can be recovered in predetermined proportions.

In its process aspects, the present invention relates to an improvement in a continuous process for converting lower olefinic hydrocarbons to $C_5^+$ liquid hydrocarbons by contacting olefinic feedstock with acid zeolite catalyst in the presence of a recycled diluent stream containing $C_3$—$C_4$ hydrocarbons in an enclosed reactor at elevated temperature and pressure. The improvement in such a process comprises the steps of a) fractionating reactor effluent in a debutanizing zone to obtain a condensed lower alkane hydrocarbon stream and a liquid $C_5^+$ hydrocarbon stream; b) splitting the liquid $C_5^+$ hydrocarbon stream into a distillate hydrocarbon range liquid product and a gasoline range overhead hydrocarbon vapor stream; c) recycling under pressure at least a portion of the condensed lower alkane hydrocarbon stream and combining said lower alkane hydrocarbon stream as a diluent with the olefinic feedstock stream; and d) recovering waste heat from the gasoline range overhead hydrocarbon vapor stream from the splitting step by passing this gasoline range overhead hydrocarbon vapor in heat exchange relationship with the combined liquid lower alkane hydrocarbon and olefinic feedstock stream. In a preferred embodiment of such an improved process, waste heat from the hot distillate range hydrocarbon liquid product can also be recovered by passing such distillate range liquid in heat exchange relationship with the combined liquid lower alkane and olefinic feedstock stream.

Typically, the olefinic feedstock for the process consists essentially of $C_2$—$C_5$ aliphatic hydrocarbons containing a major fraction of monoalkenes in the essential absence of dienes or other deleterious materials. The process may employ various volatile lower olefins as feedstock, with oligomerization of $C_2$—$C_6$ α-olefins being preferred for either gasoline or distillate production. Preferably the olefinic feedstream contains 50 to 75 mole % $C_3$—$C_5$ alkenes.

In its apparatus aspect, the present invention relates to an integrated system for converting olefinic feedstock to heavier hydrocarbons comprising gasoline range hydrocarbons and distillate range hydrocarbons by catalytic oligomerization of lower olefins at elevated temperature and pressure. Such a system comprises a) an adiabatically operated catalytic oligomerization reactor system operatively connected for serial contacting of vapor phase olefinic feedstock with a plurality of fixed aluminosilicate catalyst beds to thereby exothermically convert lower olefins to heavier hydrocarbons, with this system further comprising means for delivering pressurized liquid olefinic feedstock to the reactor system and means for removing hot reactor effluent from the reactor system; b) separator means for recovering both gasoline range hydrocarbon vapor and distillate range hydrocarbon liquid from reactor effluent; c) first heat exchange means for preheating pressurized olefinic feedstock by heat exchange with the hot gasoline range hydrocarbon vapor recovered from the reactor effluent; d) second heat exchange means for further preheating the olefinic feed stock by heat exchange with hot reactor effluent; and e) furnace means for

2

further heating the olefinic feedstock to oligomerization reaction temperature. Optionally such a system can also include additional means for preheating the olefinic feedstock by heat exchange with hot distillate range hydrocarbon liquid recovered from the separator means.

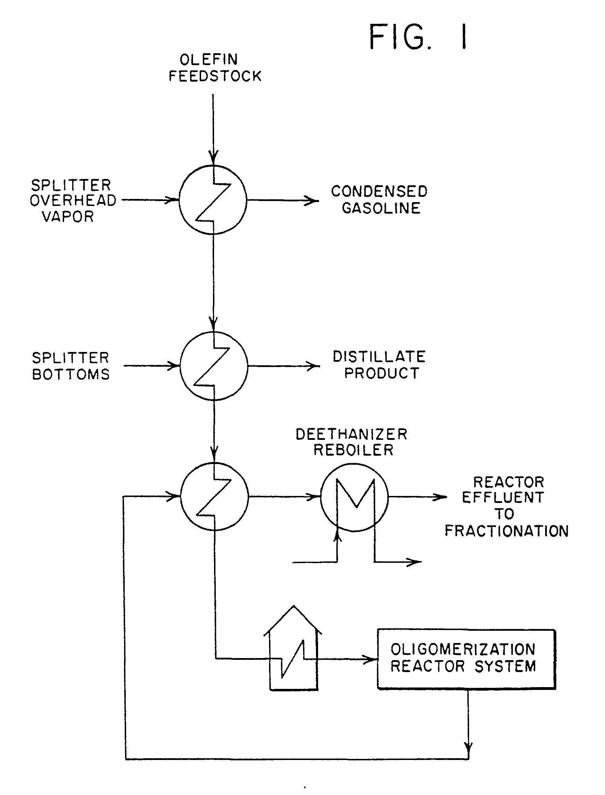

Figure 1 represents a simplified schematic drawing showing heat exchange relationships between major process streams of the present invention. In Fig. 1 an integrated system is depicted for converting olefinic feedstock to heavier hydrocarbons by catalytic oligomerization of lower olefins at elevated temperature and pressure. A series of operatively connected feedstock heating zones is provided, including first heat exchange means for preheating pressurized olefinic feedstock with hot gasoline-range vapor, thereby condensing the gasoline; second heat exchange means for heating feedstock from the first exchanger with hot distillate range liquid; third heat exchange means for heating feedstock from the second exchanger with hot oligomerization reactor effluent; and furnace means for heating feedstock from the third exchanger to reaction temperature. An adiabatically operated catalytic oligomerization reactor is provided for exothermic conversion of lower olefins to heavier hydrocarbons comprising gasoline range and distillate range hydrocarbons, followed in sequence by fluid handling means for passing hot reactor effluent from the reactor to the third exchanger to heat feedstock. Optionally, the reactor effluent thermal energy may be further recovered by heat exchange in at least one fractionator vaporization unit, such as a de-ethanizer reboiler or the like.

The continuous system also provides fractionating means for separating reactor effluent into gasoline range vapor stream, a liquid distillate range stream and a light hydrocarbon stream, and means for optionally recycling at least a portion of condensed gasoline on light hydrocarbons under pressure for combining with olefinic feedstock.

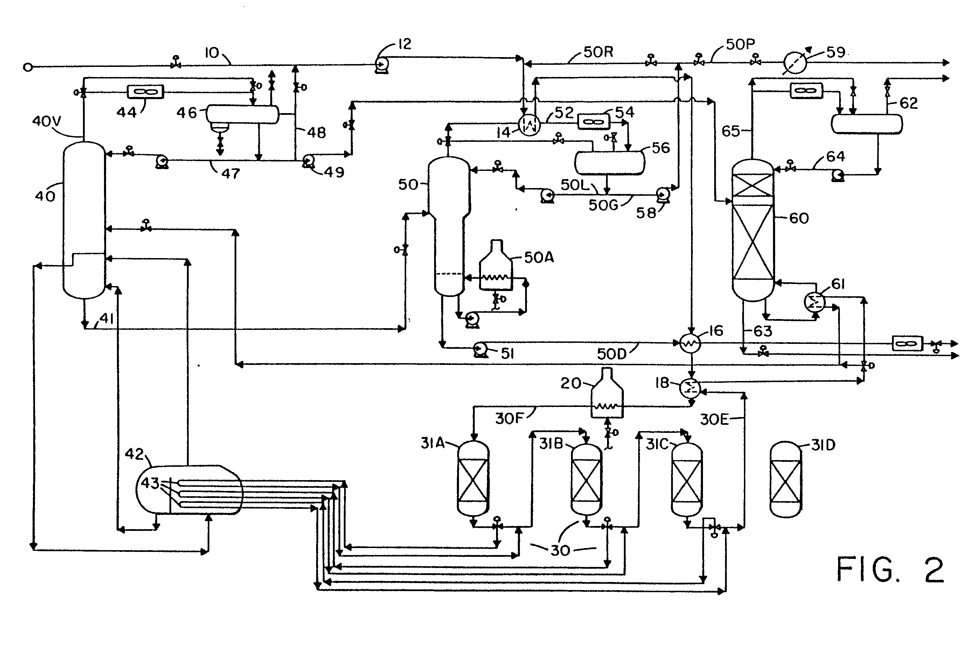

The flow diagram of Fig. 2 of the drawing represents the overall process. The olefinic feedstock is usually supplied as a liquid stream under moderate superatmospheric pressure and warm ambient temperature. Ordinarily, the feedstock is substantially below the process reactor pressure, and may be combined with recycled liquid diluent which is rich in $C_3$—$C_4$ alkanes at similar temperature and pressure. Following pressurization of the combined olefin-recycle and/or gasoline feedstreams, it is passed through the catalytic reactor system, which includes multiple fixed bed reactors operatively connected with the heat exchange system, as described later. The reactor effluent may be cooled by heat exchange with a debutanizer bottoms fraction. A condensed debutanizer overhead stream is recovered for recycle and the heavier hydrocarbons obtained by oligomerization of the feedstock is fractionated in a product splitter unit to yield a distillate fraction ($330°F^+$ [$166°C$] boiling point) and a gasoline fraction (boiling range of $125°F$ to $330°F$ [$52°C$ to $166°C$]) in varying amount.

Since the gasoline product comprises a major fraction of unsaturated aliphatic liquid hydrocarbons, it may be recovered and hydrotreated to produce spark-ignited motor fuel if desired. Optionally, all or a portion of the olefinic gasoline range hydrocarbons from the splitter unit may be recycled for further conversion to heavier hydrocarbons in the distillate range. This may be accomplished by combining the recycle gasoline with lower olefin feedstock and diluent prior to heating the combined streams.

Process conditions, catalysts and equipment suitable for use in the present invention are those given for the MOGD processes such as are described in U.S. Patents 3,960,978 (Givens et al), 4,021,502 (Plank et al), and 4,150,062 (Garwood et al). Hydrotreating and recycle of olefinic gasoline are disclosed in U.S. Patent 4,211,640 (Garwood and Lee). Other pertinent disclosures include U.S. Patent 4,227,992 (Garwood and Lee) and European Patent No. 31675 (Dwyer and Garwood) relating to catalytic processes for converting olefins to gasoline/distillate.

The catalyst materials suitable for use herein can be any acid zeolite which promotes the oligomerization of lower olefins, especially propene and butene-1, to higher hydrocarbons. The oligomerization catalysts preferred for use herein include the ZSM-5 type crystalline aluminosilicate zeolites having a silica to alumina ratio of at least 12, a constraint index of 1 to 12 and acid cracking activity of 160—200. Representative of the ZSM-5 type zeolites are ZSM-5, ZSM-11, ZSM-12, ZSM-23, ZSM-35, ZSM-38 and ZSM-48. ZSM-5 is disclosed and claimed in U.S. Patent No. 3,702,886 and U.S. Patent No. Re. 29,948; ZSM-11 is disclosed and claimed in U.S. Patent No. 3,709,979. Also, see U.S. Patent No. 3,832,449 for ZSM-12; U.S. Patent No. 4,076,842 for ZSM-23; U.S. Patent No. 4,016,245 for ZSM-35; U.S. Patent No. 4,046,839 for ZSM-38 and European Patent Publication No. 15132 for ZSM-48. One ZSM-5 type zeolite useful herein is a highly siliceous ZSM-5 described in U.S. Patent No. 4,067,724 and referred to in that patent as "silicalite".

Other catalysts which may be used in one or more reactor stages include a variety of medium pore (∿5 to 9A) siliceous materials such as borosilicates, ferrosilicates, and/or aluminosilicates disclosed in U.K. Patents 2,106,131, '132, '533 and '534. Still other effective catalysts include those zeolites disclosed in U.S. Patent 4,430,516 (Wong and LaPierre) and European Patent Application No. 83304696.4 (Koenig and Degnan), which relate to conversion of olefins over large pore zeolites.

The most preferred catalyst material for use herein is an extrudate (1.5 mm) comprising 65 weight % HZSM-5 (steamed) and 35% alumina binder, having an acid cracking activity ($\alpha$) of 160 to 200.

The process and apparatus of the present invention are illustrated in greater detail in Figure 2. Referring to Fig. 2, olefinic feedstock is supplied to the MOGD plant through liquid conduit 10 under steady stream conditions, diluted and pressurized to process pressure by pump 12. The olefinic feedstock plus recycled liquids are then sequentially heated by passing through indirect heat exchange units 14, 16, 18 and

3

furnace 20 to achieve the temperature for catalytic conversion in reactor system 30, including plural reactor vessels 31A, B, C, etc.

The reactor system section shown consists of three downflow fixed bed, series reactors on line with exchanger cooling between reactors. The reactor configuration allows for any reactor to be in any position, A, B or C.

The reactor in position A has the most aged catalyst and the reactor in position C has freshly regenerated catalyst. The cooled reactor effluent is fractionated first in a debutanizer 40 to provide lower aliphatic liquid recycle and then in splitter unit 50 which not only separates the debutanizer bottoms into gasoline and distillate products but provides liquid gasoline recycle.

The gasoline recycle is not only necessary to produce the proper distillate quality but also (with the non-olefins in the feed and $C_3$—$C_4$ lower alkane recycle) limits the exothermic rise in temperature across each reactor to less than 30°C. However, the reactor $\Delta T$'s are also a function of the $C_3$—$C_4$ recycle flow rate. Change in recycle flow rate is intended primarily to compensate for gross changes in the feed non-olefin flow rate. As a result of preheat, the liquid recycles are substantially vaporized by the time that they reach the reactor inlet.

It has been found advantageous to provide a liquid lower alkane ($C_3/C_4$) and/or gasoline recycle stream as a diluent and to combine the liquid recycle and olefin feedstock at relatively lower pressure and pump the combined feedstream up to process pressure in the liquid phase. Substantial energy savings are achieved in this technique by single stage liquid pumping with subsequent heating to vaporize the combined olefinic feedstock and diluent stream prior to catalyst contact. By pressurizing and recycling a portion of the unde-ethanized condensed lower alkane stream for diluting the olefinic feedstream at process pressure, expensive fractionation can be avoided. The de-ethanizer tower can be outside the MOGD process loop and, advantageously a further step of combining a light hydrocarbon refinery stream with the lower aliphatic stream portion prior to de-ethanizing can be included in the de-ethanizing unit.

The following is a description of the process flow in detail. Olefin feedstock under flow control is combined in conduit 10 with condensed $C_3$—$C_4$ rich recycle, which is also under flow control. The resultant stream is pumped up to system pressure by pump 12 and is combined with gasoline recycle after that stream has been pumped up to system pressure by pump 58. The combined stream (feed plus recycle plus gasoline recycle) after preheat is routed to the inlet 30F to the reactor 31A of system 30. The combined stream (herein designated as the reactor feed stream) is first preheated against the splitter tower 50 overhead in exchanger 14 (reactor feed/splitter tower overhead exchange) and then against the splitter tower bottoms in exchanger 16 (reactor feed/splitter bottoms exchanger) and then finally against the effluent from the reactor in position C, in exchanger 18 (reactor feed/reactor effluent exchanger). In the furnace 20, the reactor feed is heated to the required inlet temperature for the reactor in position A.

Because the reaction is exothermic, the effluents from the reactors in the first two positions A, B are cooled to the temperature required at the inlet of the reactors in the last two positions, B, C, by partially reboiling the debutanizer, 40. Temperature control is accomplished by allowing part of the reactor effluents to bypass the reboiler 42. Under temperature control of the bottom stage of the debutanizer, the additional required reboiling is provided by part of the effluent from the reactor 31 in position C.

After preheating the reactor feed, the reactor effluent reboils de-ethanizer bottoms 61 and is then routed as a mixed phase stream $80^+$% vapor to the debutanizer which is operated at a pressure which completely condenses the debutanizer tower overhead 40V by cooling in condenser 44. The liquid from debutanizer overhead accumulator 46 provides the tower reflux 47, the lower alkane recycle 48 and feed to the de-ethanizer 60, which, after being pumped to the de-ethanizer pressure by pump 49 is sent to the de-ethanizer 60. The de-ethanizer accumulator overhead 65 is routed to the fuel gas system 62. The accumulator liquid 64 provides the tower reflux. The bottoms stream 63 (LPG product) may be sent to an unsaturated gas plant or otherwise recovered.

The bottoms stream 41 from the debutanizer 40 is sent directly to the splitter 50 which splits the $C_5^+$ material into $C_5$—330°F ($C_5$—166°C) gasoline (overhead liquid product and recycle) and 330°F$^+$ (166°C$^+$) distillate (bottoms product). The splitter tower overhead stream 52, after preheating the reactor feed stream is totally condensed in the splitter tower overhead condenser 54. The liquid from the overhead accumulator 56 provides the tower reflux 50L, the gasoline product 50P and the specified gasoline recycle 50R under flow control. For example, 1 mole gasoline/mole olefin in feed is pressurized by pump 58 for recycle. After being cooled in the gasoline product cooler 59, the gasoline product is sent to the gasoline pool. The splitter bottoms fraction is pumped to the required pressure by pump 51 and then preheats the reactor feed stream in exchanger 16. Finally, the distillate product 50D is cooled to ambient temperature before being hydrotreated to improve its cetane number.

From an energy conservation standpoint, it is advantageous to reboil the debutanizer using all three reactor effluents as opposed to using a fired reboiler. A kettle reboiler 42 containing 3 U-tube exchangers 43 in which the reactor 31 effluents are circulated is a desirable feature of the system. Liquid from the bottom stage of debutanizer 40 is circulated in the shell side. Alternatively three thermosyphon reboilers in series would suffer the disadvantages of a large pressure drop and control problems inherent in the instability resulting from the tower bottoms being successively vaporized in each reboiler. Although the pressure drop problem would be overcome with three reboilers in parallel, there would be considerable difficulty in controlling the allocation of tower bottoms to each parallel reboiler.

4

**0 127 284**

In order to provide the desired quality and rate for both liquid lower alkane (C₃—C₄) and gasoline recycles, it is necessary to fractionate the reactor effluent. Phase separators do not give the proper separation of the reactor effluent to meet the quality standards and rate for both liquid recycles. For example, the gasoline recycle would carry too much distillate and lights, while the C₃—C₄ recycle would contain gasoline boiling cuts. Consequently, it would be difficult to properly control the liquid recycles if separators were employed.

The fractionation sub-system has been devised to yield three main liquid product streams—LPG (mainly C₃—C₄ alkanes), gasoline boiling range hydrocarbons (C₅ to 330°F) [C₅ to 166°C] and distillate range heavier hydrocarbons (330°F⁺) [166°C⁺]. De-ethanizer off gas comprising methane and ethane with minor amounts of other light gases may be consumed within the MOGD system as furnace fuel gas, flared, or otherwise utilized.

While conventional refinery practice in fractionating hydrocarbon streams first provides for de-ethanizing the stream, followed by debutanizing and product splitting in sequence; it has been found to be advantageous in the present system to effect an initial fractionation of the entire MOGD reactor effluent to provide a light stream (C₄⁻) and a normally liquid C₅⁺ product stream. Ordinarily the reactor effluent is introduced to the initial fractionation unit as a mixed phase stream.

By operating the debutanizer unit at adequate pressure to condense the overhead C₄⁻ vapors a liquid recycle stream can be fed to the MOGD reactor system with olefin feedstock. This condensed liquid stream can contain lower C₁—C₂ components as well as the liquid C₃—C₄ components, thus obviating the need for further fractionation of a significant portion of the de-butanizer overhead.

By placing the de-ethanizer unit outside the MOGD recycle loop, the cost of fractionating the offstream into liquid petroleum gas (LPG) and off gas can be reduced. The de-ethanizer function need not be dedicated to the olefins oligomerization plant, but may be integrated with other refinery streams. Optionally, one or more light gas straems containing C₁ to C₄ aliphatic hydrocarbons may be combined with a non-recycled portion of the de-butanizer overhead for economic separation and recovery of the components. Existing de-ethanizing capacity may be employed, where the fractionation streams are compatible. Various heat exchange schemes are feasible within the inventive concept.

The de-ethanizer fractionation unit 60 may be a tray-type design or packed column, with about 13 to 18 theoretical stages being provided for optimum LPG product. With proper feedtray locations between 3 and 7 trays from the top, 15 theoretical stages in the de-ethanizer are adequate to assure proper fractionation. The deethanizer tower diameter, related fractionation equipment and heat exchange area are reduced considerably from conventional systems by reason of the prior debutanizing and withdrawing condensed light hydrocarbon for recycle. Since the deethanizer unit is operated at a significantly higher pressure (e.g. 10—15 atmospheres) than the debutanizer or splitter columns, pumping energy is significantly reduced with a small mass flow rate.

The product splitter fractionation unit 50 receives the debutanizer bottoms, preferably as a mixed phase stream containing a major fraction of vapor (e.g. 70 weight %). The main splitter column may be a tray-type or packed vertical fractionating column, with a furnace fixed bottoms reboiler 50A and gasoline reflux loop 14, 52, 54, 56, 50B. The splitter distillation tower 50 is preferably operated at substantially atmospheric pressure to avoid excessive bottoms temperature, which might be deleterious to the distillate product. The fractionation equipment and operating techniques are substantially similar for each of the major stills 40, 50, 60, with conventional plate design, reflux and reboiler components. The fractionation sequence and heat exchange features of the present system are operatively connected in an efficient MOGD system provide significant economic advantages.

By comparison with conventional fractionation systems wherein the entire reactor effluent would be first de-ethanized and then debutanized and split into C₅⁺ gasoline or distillate fractions; the present fractionation system requires fewer overall theoretical stages in the total distillation tower complex.

MOGD operating modes may be selected to provide maximum distillate product by gasoline recycle and optimal reactor system conditions; however, it may be desired to increase the output of gasoline by decreasing or eliminating the gasoline recycle. Operating examples are given for both the distillate mode and gasoline mode of operation, utilizing as the olefinic feedstock a pressurized stream FCC olefinic effluent (about 1200 kPa) comprising a major weight and mole fraction of C₃⁼/C₄⁼, as set forth in Table I. The adiabatic exothermic oligomerization reaction conditions are readily optimized at elevated temperature and/or pressure to increase distillate yield or gasoline yield as desired, using H-ZSM-5 type catalyst. Particular process parameters such as space velocity, maximum exothermic temperature rise, etc. may be optimized for the specific oligomerization catalyst employed, olefinic feedstock and desired product distribution.

A typical distillate mode multi-zone reactor system employs inter-zone cooling, whereby the reaction exotherm can be carefully controlled to prevent excessive temperature above the normal moderate range of 190° to 315°C (375°—600°F).

Advantageously, the maximum temperature differential across any one reactor is about 30°C (Δ T∾50°F) and the space velocity (LHSV based on olefin feed) is 0.5 to 1. Heat exchangers provide inter-reactor cooling and reduce the effluent to fractionation temperature. It is an important aspect of energy conservation in the MOGD system to utilize at least a portion of the reactor exotherm heat value by exchanging hot reactor effluent from one or more reactors with a fractionator stream to vaporize a liquid

5

## 0 127 284

hydrocarbon distillation tower stream, such as the debutanizer reboiler. Optional heat exchangers may recover heat from the effluent stream prior to fractionation. Gasoline from the recycle conduit is pressurized by pump means and combined with feedstock, preferably at a mole ratio of 1—2 moles per mole of olefin in the feedstock.

It is preferred to operate in the distillate mode at elevated pressure of 4200 to 7000 kPa (600—1000 psig). A typical material balance for distillate mode operation is given in Table I.

## TABLE I
### Stream components
### Mole %—Distillate mode

| Component | Feedstock (fresh olefins) | Liquid $C_3$—$C_4$ recycle | Gasoline recycle/product | Reactor feedstream | Reactor effluent | Debutanizer bottoms | De-ethanizer overhead | De-ethanizer reflux | De-ethanizer off-gas (fuel) | De-ethanizer bottoms (LPG) |
|---|---|---|---|---|---|---|---|---|---|---|
| $C_1$ | 0 | 0.27 | 0 | .04 | .12 | 0 | .76 | .32 | 3.39 | 0 |
| $C_2^=$ | .12 | .13 | 0 | .08 | .06 | 0 | .68 | .51 | 1.66 | 0 |
| $C_2$ | 1.04 | 2.52 | 0 | .88 | 1.15 | 0 | 16.54 | 13.95 | 32.08 | 0 |
| $C_3^=$ | 31.93 | 3.47 | 0 | 15.70 | 1.58 | 0 | 11.48 | 11.66 | 10.40 | 2.88 |
| $C_3$ | 11.98 | 29.92 | 0 | 10.25 | 13.61 | 0 | 61.12 | 63.16 | 48.9 | 28.27 |
| $iC_4$ | 17.61 | 40.34 | .22 | 14.60 | 18.46 | .20 | 7.26 | 7.99 | 2.85 | 43.54 |
| $C_4^=$ | 31.81 | 10.36 | .15 | 16.75 | 4.78 | .13 | 1.23 | 1.37 | .43 | 11.21 |
| $nC_4$ | 4.80 | 12.49 | .54 | 4.38 | 5.94 | .47 | .92 | 1.03 | .28 | 13.53 |
| $iC_5$ | .39 | .34 | 10.64 | 4.20 | 5.31 | 9.36 | 0 | 0 | 0 | .37 |
| $C_5^=$ | .30 | .17 | 9.56 | 3.72 | 4.65 | 8.4 | 0 | 0 | 0 | .18 |
| $nC_5$ | .01 | 0 | .52 | .20 | .25 | .46 | 0 | 0 | 0 | 0 |
| Gasoline | 0 | 0 | 75.38 | 28.08 | 36.3 | 66.62 | 0 | 0 | 0 | 0 |
| Distillate | 0 | 0 | 2.99 | 1.11 | 7.83 | 14.37 | 0 | 0 | 0 | 0 |
| $H_2O$ | .01 | 0 | 0 | .01 | .01 | 0 | 0 | 0 | 0 | 0 |
| Mass flow | 100 | 33.3 | 160.4 | 293.7 | 293.7 | 212.6 | 21.3 | 18.5 | 2.8 | 45.1 |
| Stream No. (Fig. 2) | 10 | 48 | 50G | 30F | 30E | 41 | 65 | 64 | 62 | 63 |

The mass flow rate relative to the major process streams for a preferred distillate-optimized MOGD plant are given in Table II, along with process temperature and pressure conditions. The mass flow rate at steady state is expressed in part by weight per 100 parts of fresh feed.

TABLE II

| Process stream/No. | Mass flow rate | Temperature (°C) | Pressure kPa(a) (kilo Pascals) absolute |
|---|---|---|---|
| Feedstock/10 | 100 | 38 | 1205 |
| C₃—C₄ recycle/48 | 33.3 | 43 | 1010 |
| Gasoline recycle/59 | 160.4 | 65 | — |
| Reactor feed/30F | 293.7 | 232/271* | 4200 |
| Reactor effluent/30E | 293.7 | 236/259* | 3686 |
| Debut. overhead/40V | 183.9 | 61 | 1050 |
| Debut. reflux/47 | 102.9 | — | 1015 |
| Debut. over. prod./48 | 81.1 | 43 | 1015 |
| Debut. bottoms/41 | 212.6 | 197 | 1100 |
| Deeth. feed/60F | 47.8 | 43 | 2140 |
| Deeth. overhead/65 | 21.3 | 58 | 2100 |
| Deeth. reflux/64 | 18.5 | 43 | — |
| Deeth. off gas/62 | 2.8 | 43 | 2070 |
| LPG Prod./63 | 45.1 | 91 | 2110 |
| Splitter overhead/52 | 196.6 | 124 | 160 |
| Splitter reflux/50B | 28.3 | 65 | 105 |
| Splitter product/50G | 168.3 | 65 | 105 |
| Gasoline product/50P | 8 | 43 | 790 |
| Distillate product/50D | 44.3 | 43 | 970 |

\* Start of cycle (SOC)/End of cycle (EOC)

The gasoline product is recovered from this mode of operation at the rate of 8% of olefinic feedstock, whereas distillate is recovered at 44% rate. Product properties are shown in Table III.

TABLE III
Product properties

| Properties | Gasoline $C_6$—330°F | Distillate 330°F+ (RAW) |
|---|---|---|
| Gravity, °API | 62.8 | 48.5 |
| Total sulfur, ppmw | 0 | 0 |
| Octane number, R+O | 90 | — |
| Bromine number | — | 78.9 |
| Weight % $H_2$ | — | 14.3 |
| Aniline Pt | — | 163 |
| Freeze Pt (°F) | — | <−76 |
| Cetane number | — | 33 |
| Luminometer number | — | 69 |
| **ASTM Distillation** | **D-86** | **D-1160** |
| IBP | 165 | 348 |
| 10/30 | 217/252 | 379/407 |
| 50/70 | 284/316 | 449/511 |
| 90 | 414 | 676 |
| 95 | — | 770 |
| EP | 531 | |

The reactor system contains multiple downflow adiabatic catalytic zones in each reactor vessel. The liquid hourly space velocity (based on total fresh feedstock) is about 1 LHSV. In the distillate mode the inlet pressure to the first reactor is about 4200 kPa (600 psig total), with an olefin partial pressure of at least about 1200 kPa. Based on olefin conversion of 50% for ethene, 95% for propene, 85% for butene-1 and 75% for pentene-1, and exothermic heat of reaction is estimated at 450 BTU per pound (1047 kJ/kg) of olefins converted. When released uniformly over the reactor beds, a maximum ΔT in each reactor is about 30°C. In the distillate mode the molar recycle ratio for gasoline is equimolar based on olefins in the feedstock, and the $C_3$—$C_4$ molar recycle is 0.5:1.

From the olefinic feedstock, which contains about 62% olefins, the distillate mode operation described produces about 31 vol. % distillate along with about 6.3% gasoline, 6% LPG and 38+% unconverted olefins and saturated aliphatics in the feed.

By way of comparison, the distillate mode is compared with operation of the same system shown in Fig. 2, except that the reactor system is operated at relatively elevated temperature and moderate pressure with no gasoline recycle. The distillate yield is reduced to about 13 vol. % and the gasoline yield increased to about 27%.

The gasoline mode reactor is operated at the higher conversion temperature and does not require maximum differential temperature control closer than about 65°C (ΔT∿120°F) in the approximate elevated range of 230° to 375°C (450°—700°F). The reactor bed is maintained at a moderate superatmospheric pressure of 400 to 3000 kPa (50—400 psig), and the space velocity for ZSM-5 catalyst to optimize gasoline production should be 0.5 to 2 (LHSV). Preferably, all of the catalyst reactor zones in the system comprise a fixed bed down flow pressurized reactor having a porous bed of ZSM-5 type catalyst particles with an acid activity of 160 to 200, identical with the distillate mode system for simplifying mode selection and cyclic operation.

By comparison with the distillate mode examples, the gasoline mode system is operated at the same space velocity (LHSV=1, based on total fresh feed), maximum allowable temperature rise (ΔT∿28°C), catalyst aging rates and elevated temperature (SOC=230°C min., EOC=295°C max.). Total reactor pressure is reduced to 2160 kPa (300 psig), with a minimum olefin partial pressure at reactor inlet of about 350 kPa (50 psia). In the gasoline mode the exothermic heat of reaction is reduced from 450 BTU/pound (1047 kJ/kg) to 380 BTU/pound (884 kJ/kg) of olefins converted. Since the gasoline recycle is reduced from equimolar amounts with the olefins to nil, the $C_3$—$C_4$ recycle mol ratio is increased from 0.5:1 to 2:1 to provide adequate diluent. Under the stated gasoline mode conditions ethylene conversion is about 50%, propene, 95%; butene-1, 85%; and pentene-1, 75%. On a weight percent basis the gasoline ($C_6$—330°F) [$C_6$—166°C] yield is 52.4% with 32% distillate (330°F$^+$) [166°C$^+$], as compared to 12.6 weight % and 79%, respectively in the distillate mode.

Heat integration and fractionation techniques may be adapted to accommodate optional distillate or gasoline modes. The combined olefin/$C_3$—$C_4$ recycle feedstream may be preheated by debutanizer bottoms in an optional exchanger. Additional pump capacity may be required to handle increased recycle liquid.

Preferably the ZSM-5 catalyst is kept on stream until the coke content increases from 0% at the start of cycle (SOC) until it reaches a maximum of 30 weight % at end of cycle (EOC) at which time it is regenerated by oxidation of the coke deposits. Typically a 30-day total cycle can be expected between regenerations. The reaction operating temperature depends upon its serial position. The system is operated advantageously (as shown in Fig. 2) by increasing the operating temperature of the first reactor (Position A) from 230°C—255°C (SOC) to 270°C—295°C (EOC) at a catalyst aging rate of 3—6°C/day. Reactors in the second and subsequent positions (B, C, etc.) are operated at the same SOC temperature; however, the lower aging rate (e.g. −3°C/day) in continuous operation yields a lower EOC maximum temperature (e.g.—about 275°C), after about 7 days on stream. The end of cycle is signalled when the outlet temperature of the reactor in position A reaches its allowable maximum. At this time the inlet temperature is reduced to start of cycle levels in order to avoid excessive coking over the freshly regenerated catalyst when reactor 31D is brought on-line, after having been brought up to reaction pressure with an effluent slip stream.

Regeneration of coked catalyst may be effected by any of several procedures. The catalyst may be removed from the reactor of the regeneration treatment to remove carbonaceous deposits or the catalyst may be regenerated in-situ in the reactor.

It is preferred to have at least three adiabatic reactors in continuous service; however, the T becomes smaller with increased numbers of serial reactors and difficulties may be encountered in exploiting the reaction exotherm for reboiling the debutanizer unit and preheating reactor feed. A smaller number of serial reactors in the system would require much greater $C_3$—$C_4$ recycle to control the reaction exotherms from catalytic oligomerization.

Individual reactor vessels should be sized to accommodate the fixed catalyst bed with a normal pressure drop of about 100 kPa (15 psi) and total mass flow rate of about 3600 lbs/hr-ft$^2$ (17577 kg/hr-m$^2$). A typical vessel is constructed of steel or steel alloy to withstand process pressure up to about 70 atmospheres (7000 kPa) at maximum operating temperature. An enclosed cylindrical vessel with L/D ratio of 2:1—10:1, preferably 4:1 to 6:1, is satisfactory. Since the reactor feed stream is completely vaporized or contains a minor amount of hydrocarbon liquid, no special feed distributor internal structure is required to obtain substantially uniform downward flow across the catalyst bed.

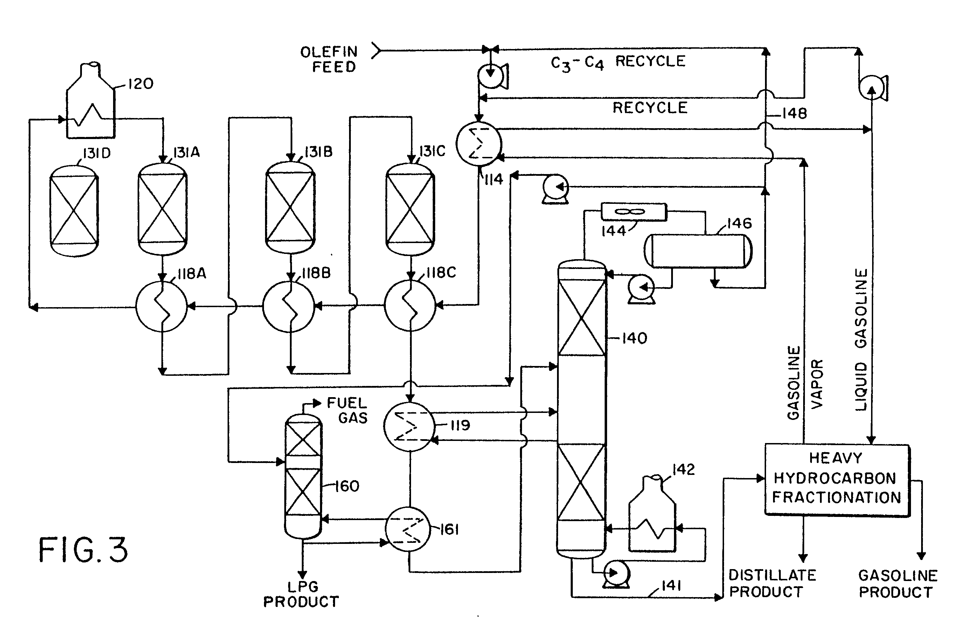

An alternative technique for operating an MOGD plant is shown in Fig. 3, which employs $C_3$—$C_4$ recycle 148 for diluting the olefin feedstock. The combined reactor feedstream is heated indirectly by fractionator overhead gasoline vapor in exchanger unit 114 and passed sequentially through reactor effluent exchangers 118C, 118B, 118A and furnace 120 before entering catalytic reactors 131A, B, C. Heat is exchanged between debutanizer 140 and hot reactor effluent in exchanger 119 to vaporize a lower tower fraction rich in $C_5^+$ hydrocarbons. The debutanizer bottoms are withdrawn through $C_5^+$ product line 141 and reboiled by furnace 142. Light gases from the debutanizer 140 are condensed in air cooler 144 and separated in accumulator 146 for reflux and recycle. A portion of the condensed light hydrocarbon stream is deethanized in tower 160 to provide fuel off gas and LPG product. The liquid from the bottom stage is reboiled by reactor effluent in exchanger 161 to recover additional heat values and to partially condense the heavier hydrocarbon in the effluent prior to debutanizing.

**Claims**

1. In the continuous process for converting lower olefinic hydrocarbons to $C_5^+$ liquid hydrocarbons by contacting olefinic feedstock with acid zeolite catalyst in the presence of a recycled diluent stream containing $C_3$—$C_4$ hydrocarbons in an enclosed reactor at elevated temperature and pressure, the improvement which comprises:

a) fractionating reactor effluent in a debutanizing zone to obtain a condensed lower alkane hydrocarbon stream and a liquid $C_5^+$ hydrocarbon stream;

b) splitting the liquid $C_5^+$ hydrocarbon stream into a distillate range hydrocarbon liquid product and a gasoline range overhead hydrocarbon vapor stream;

c) recycling under pressure at least a portion of said condensed lower alkane hydrocarbon stream and combining said lower alkane hydrocarbon stream as a diluent with said olefinic feedstock stream; and

d) recovering waste heat from said gasoline range overhead hydrocarbon vapor stream from the splitting step by passing said gasoline range overhead hydrocarbon vapor in heat exchange relationship with said condensed liquid lower alkane hydrocarbon and olefinic feedstock stream.

2. A process according to Claim 1 which comprises the additional step of further recovering waste heat from the hot distillate range hydrocarbon liquid product from the splitting step by passing said distillate range hydrocarbon liquid product in heat exchange relationship with said combined liquid lower alkane hydrocarbon and olefinic feedstock stream.

3. A process according to Claim 1 or Claim 2 wherein a portion of the gasoline stream is recycled and combined with liquid olefinic feedstock and lower alkane diluent to further react olefinic gasoline components at elevated pressure and moderate temperature and to thereby increase distillate yield.

4. A process according to Claim 1 or Claim 2 wherein substantially all gasoline range hydrocarbons are recovered from the process as product without substantial recycle thereof and wherein the catalytic reactor is operated at elevated temperature and moderate pressure to increase gasoline yield.

5. A process according to any of Claims 1 to 4 which comprises the additional step of fractionating at least a portion of the condensed lower alkane hydrocarbon stream in a de-ethanizing zone to produce a $C_3$—$C_4$ LPG product and a $C_2^-$ gaseous overhead product.

6. A process according to any of Claims 1 to 5 which comprises the additional steps of recovering heat from the reactor effluent by passing said effluent in heat exchange relationship with said combined stream of olefinic feedstock and recycled lower alkane hydrocarbon diluent.

7. A process according to any of Claims 1 to 6 wherein the recycled lower alkane contains at least 80 mole % $C_3$—$C_4$ alkanes and is combined with olefinic feedstream at a mole ratio of 0.5:1 to 2:1, based on olefin in fresh feed.

8. An integrated system for converting olefinic feedstock to heavier hydrocarbons comprising gasoline range hydrocarbons and distillate range hydrocarbons by catalytic oligomerization of lower olefins at elevated temperature and pressure, said system comprising:

a) an adiabatically operated catalytic oligomerization reactor system operatively connected for serial contacting of vapor phase olefinic feedstock with a plurality of fixed aluminosilicate catalyst beds to thereby exothermically convert lower olefins to heavier hydrocarbons, said system further comprising means for delivering pressurized liquid olefinic feedstock to said reactor system and means for removing hot reactor effluent from said reactor system;

b) separator means for recovering both gasoline range hydrocarbon vapor and distillate range hydrocarbon liquid from reactor effluent;

c) first heat exchange means for preheating pressurized olefinic feedstock by heat exchange with said hot gasoline range hydrocarbon vapor recovered from said reactor effluent;

d) second heat exchange means for further preheating said olefinic feed stock by heat exchange with hot reactor effluent; and

e) furnace means for further heating said olefinic feedstock to oligomerization reaction temperature.

9. A system according to Claim 8 which further comprises additional means for preheating said pressurized liquid olefinic feedstock by heat exchange with hot distillate range hydrocarbon liquid recovered from said reactor effluent.


## Patentansprüche

1. In einem kontinuierlichen Verfahren zur Umwandlung niederer olefinischer Kohlenwasserstoffe zu flüssigen $C_5^+$-Kohlenwasserstoffen durch Kontakt des olefinischen Ausgangsproduktes mit einem sauren Zeolithkatalysator in Gegenwart eines rezirkulierten Verdünnerstromes, der $C_3$—$C_4$-Kohlenwasserstoffe enthält, in einem abgeschlossenen Reaktor bei erhöhter Temperatur und Druck umfaßt die Verbesserung:

a) Fraktionierung des Reaktorabstromes in einer Debutanisierzone, um einen kondensierten Kohlenwasserstoffstrom niederer Alkane und einen flüssigen $C_5^+$-Kohlenwasserstoffstrom zu erhalten,

b) Trennung des flüssigen $C_5^+$-Kohlenwasserstoffstromes in ein flüssiges Kohlenwasserstoffprodukt vom Destillatbereich und einen Kohlenwasserstoff-Kopfproduktdampfstrom im Benzinbereich,

c) Rezirkulieren zumindest eines Teils des kondensierten Kohlenwasserstoffstromes niederer Alkane unter Druck und Kombinieren dieses Kohlenwasserstoffstromes niederer Alkane als Verdünner mit dem olefinischen Ausgangsprodukt-Strom.

d) Rückgewinnung der Abwärme aus dem Kohlenwasserstoff-Kopfproduktdampfstrom im Benzinbereich aus dem Trennungsschritt, indem der Kohlenwasserstoff-Kopfproduktdampfstrom im Benzinbereich im Wärmeaustauschverhältnis mit dem kondensierten flüssigen Kohlenwasserstoffstrom niederer Alkane und dem olefinschen Ausgangsprodukt-Strom geführt wird.

2. Verfahren nach Anspruch 1, welches den zusätzlichen Schritt der zweiteren Rückgewinnung von Abwärme aus dem heißen flüssigen Kohlenwasserstoffprodukt im Destillatbereich aus dem Trennungsschritt umfaßt, indem dieses flüssige Kohlenwasserstoffprodukt im Destillatbereich im Wärmeaustauschverhältnis mit dem kombinierten flüssigen Kohlenwasserstoffstrom niederer Alkane und dem olefinischen Ausgangsprodukt-Strom geführt wird.

3. Verfahren nach Anspruch 1 oder 2, worin ein Teil des Benzinstromes rezirkuliert wird und mit dem flüssigen olefinischen Ausgangsprodukt und dem Verdünner niederer Alkane kombiniert wird, damit die olefinischen Benzinkomponenten bei erhöhtem Druck und gemäßigter Temperatur weiter reagieren und dadurch die Destillatausbeute erhöht wird.

4. Verfahren nach Anspruch 1 oder 2, worin im wesentlichen alle Kohlenwasserstoffe des Benzinbereiches aus dem Verfahren im wesentlichen ohne deren Kreislaufführung als Produkt zurückgewonnen werden und worin der katalytische Reaktor bei erhöhter Temperatur und gemäßigter Temperatur betrieben wird, um die Benzinausbeute zu erhöhen.

5. Verfahren nach einem der Ansprüche 1 bis 4, welches den zusätzlichen Schritt der Fraktionierung zumindest eines Teils des kondensierten Kohlenwasserstoffstromes niederer Alkane in einer Deethanisierungs-Zone umfaßt, um ein $C_3$—$C_4$-LPG-Produkt und ein gasförmiges $C_2^-$-Kopfprodukt zu erzeugen.

6. Verfahren nach einem der Ansprüche 1 bis 5, welches die zusätzlichen Schritte der Wärmerückgewinnung aus dem Reaktorabstrom umfaßt, in dem der Abstrom im Wärmeaustauschverhältnis mit dem kombinierten Strom des olefinischen Ausgangsproduktes und des rezirkulierten Kohlenwasserstoffverdünners niederer Alkane geführt wird.

7. Verfahren nach einem der Ansprüche 1 bis 6, worin das rezirkulierte niedere Alkan mindestens 80 Mol-% $C_3$—$C_4$-Alkane enthält und mit dem olefinischen Zufuhrstrom bei einem Molverhältnis von 0,5:1 bis 2:1 bezogen auf das Olefin in der frischen Zufuhr kombiniert wird.

8. Integriertes System zur Umwandlung eines olefinischen Ausgangsproduktes zu schwereren Kohlenwasserstoffen, die Kohlenwasserstoffe des Benzinbereiches und Kohlenwasserstoffe des Destillatbereiches umfassen, durch katalytische Oligomerisierung niederer Olefine bei erhöhter Temperatur und Druck, wobei das System umfaßt:

a) ein adiabatisch betriebenes katalytisches Oligomerisierungs-Reaktorsystem, das zum reihenweisen Kontakt der Dampfphase des olefinischen Ausgangsproduktes mit einer Vielzahl von Aluminosilicat-Katalysatorfestbetten wirksam verbunden ist, um dadurch niederer Olefine exotherm zu schwereren Kohlenwasserstoffen umzuwandeln, wobei das System weiterhin eine Einrichtung, um komprimiertes flüssiges olefinisches Ausgangsprodukt dem Reaktorsystem zuzuführen, und eine Einrichtung umfaßt, um heißen Reaktorabstrom aus dem Reaktorsystem zu entfernen,

b) eine Trennungseinrichtung zur Rückgewinnung sowohl des Kohlenwasserstoffdampfes im Benzinbereich als auch der Kohlenwasserstoffflüssigkeit im Destillatbereich aus dem Reaktorabprodukt,

c) eine erste Wärmeaustauscheinrichtung zum Vorheizen des komprimierten olefinischen Ausgangsproduktes durch Wärmeaustausch mit dem heißen Kohlenwasserstoffdampf im Benzinbereich, der aus dem Reaktorabstrom zurückgewonnen wurde,

d) eine zweite Wärmeaustauscheinrichtung zum weiteren Vorheizen des olefinischen Ausgangsproduktes durch Wärmeaustausch mit dem heißen Rekatorabstrom und

e) eine Ofeneinrichtung zum weiteren Erwärmen des olefinischen Ausgangsproduktes auf die Reaktionstemperatur der Oligomerisierung.

9. System nach Anspruch 8, das weiterhin eine zusätzliche Einrichtung umfaßt, um das komprimierte flüssige olefinische Ausgangsprodukt durch Wäreaustausch mit der heißen Kohlenwasserstoffflüssigkeit des Destillatbereiches vorzuheizen, die aus dem Reaktorabstrom zurückgewonnen wurde.

## Revendications

1. Dans le procédé en continu de conversion des hydrocarbures oléfiniques inférieurs en hydrocarbures liquides en $C_5^+$ par mise au contact de la charge d'alimentation oléfinique avec un catalyseur à base de zéolite acide, en présence d'un courant diluant de recyclage contenant des hydrocarbures en $C_3$—$C_4$ dans un réacteur fermé, à température et pression élevées, l'amélioration qui comprend:

a) le fractionnement de l'effluent du réacteur dans une zone de séparation de butane pour obtenir un courant d'hydrocarbures à base d'alcanes inférieurs condensés et un courant d'hydrocarbures liquides en $C_5^+$;

b) fractionnement du courant d'hydrocarbures liquides en $C_5^+$ en un produit liquide hydrocarbone constituant le distillat et un courant de vapeur d'hydrocarbures de tête formant l'essence;

c) recyclage sous pression d'au moins une partie de ce courant d'hydrocarbures condensés à base d'alcanes inférieurs et combinaison de ce courant d'hydrocarbures à base d'alcanes inférieurs servant de diluant avec ladite charge d'alimentation oléfinique;

d) récupération de la chaleur transportée par ce courant de vapeur d'hydrocarbures de tête constituant l'essence par une étape de fractionnement en faisant passer cette vapeur d'hydrocarbures de tête constituant l'essence dans un échangeur de chaleur au contact duquel passe le courant d'hydrocarbures condensés à base d'alcanes inférieurs liquides et le courant de la charge d'alimentation oléfinique.

2. Un procédé selon la revendication 1, qui comprend une étape supplémentaire dans laquelle on récupère la chaleur transportée par le produit liquide chaud d'hydrocarbures constituant le distillat en le faisant passer dans un échangeur de chaleur au contact duquel se réchauffent les hydrocarbures liquides à base d'alcanes inférieurs et le charge oléfinique réunis.

3. Un procédé selon l'une ou l'autre des revendications 1 ou 2, dans lequel une partie du courant d'essence est recyclée et réunie à la charge d'alimentation liquide et au diluant à base d'alcanes inférieurs pour réagir ensuite avec les composants de l'essence oléfinique à pression élevée et à température modérée et pour accroître ainsi le rendement en distillat.

4. Un procédé selon l'une ou l'autre des revendications 1 ou 2, dans lequel pratiquement tous les hydrocarbures constituant l'essence sont récupérés dans le procédé comme sous-produits, sans les recycler de façon importante, et dans lequel le réacteur à catalyse fonctionne à température élevée et à pression modérée pour augmenter le rendement en essence.

5. Un procédé selon l'une quelconque des revendications 1 à 4, qui comprend l'étape additionnelle de fractionnement d'une portion au moins du courant d'hydrocarbures condensés à base d'alcanes inférieurs dans une tour de séparation d'éthane pour produire un produit LPG en $C_3$—$C_4$ et un produit gazeux de tête en $C_2^-$.

6. Un procédé selon l'une quelconque des revendications 1 à 5, qui comprend les étapes supplémentaires de récupération de chaleur à partir de l'effluent du réacteur en faisant passer cet effluent dans un échangeur de chaleur au contact duquel se réchauffent le courant réuni de la charge oléfinique et le diluant d'hydrocarbures à base d'alcanes inférieurs de recyclage.

7. Un procédé selon l'une quelconque des revendications 1 à 6, dans lequel l'alcane inférieur de recyclage contient au moins 80% en mole d'alcanes en $C_3$—$C_4$ et est réuni avec le courant oléfinique d'alimentation dans un rapport molaire de 0,5/1 à 2/1 par rapport à l'oléfine dans la charge fraîche.

8. Un système intégré de conversion de la charge d'alimentation oléfinique en hydrocarbures plus lourds comprenant les hydrocarbures constituant l'essence et les hydrocarbures constituant le distillat par oligomérisation catalytique d'oléfines inférieures à température et pression élevées, ce système comprenant:

a) un système de réacteur pour oligomérisation catalytique réalisée adiabatiquement, relié en fonctionnement à plusieurs lits fixes de catalyseur d'aluminosilicate au contact desquels se trouve successivement la charge oléfinique en phase vapeur, système destiné à convertir avec dégagement de chaleur les oléfines inférieures en hydrocarbures plus lourds, ce système comprenant en outre des moyens pour introduire dans ce système de réacteur la charge d'alimentation oléfinique liquide sous pression et des moyens pour enlever l'effluent chaud du réacteur de ce système de réacteur;

b) des moyens de séparation pour récupérer à la fois la vapeur d'hydrocarbures constituant l'essence et le liquide à base d'hydrocarbures constituant le distillat de l'effluent du réacteur;

c) un premier échangeur pour préchauffer la charge oléfinique sous pression par échange de chaleur avec cette vapeur d'hydrocarbures chaude constituant l'essence, récupérée dans l'effluent de ce réacteur;

d) un second échangeur pour préchauffer encore cette charge d'alimentation oléfinique par échange de chaleur avec l'effluent chaud du réacteur; et

e) un four pour chauffer encore cette charge d'alimentation oléfinique à la température de la réaction d'oligomérisation.

9. Un système selon la revendication 8, qui comprend encore des moyens supplémentaires de préchauffage de cette charge d'alimentation oléfinique liquide sous pression par échange de chaleur avec le liquide chaud à base d'hydrocarbures constituant le distillat, récupéré dans l'effluent de ce réacteur.

# FIG. 1

OLEFIN
FEEDSTOCK

SPLITTER
OVERHEAD →
VAPOR

CONDENSED
GASOLINE

SPLITTER →
BOTTOMS

DISTILLATE
PRODUCT

DEETHANIZER
REBOILER

REACTOR
EFFLUENT
TO
FRACTIONATION

OLIGOMERIZATION
REACTOR SYSTEM

1

FIG. 2

FIG.3